# EUROPEAN PATENT SPECIFICATION

(11) **EP 1 205 754 B1**
(45) Date of publication and mention of the grant of the patent: **13.07.2005**
(21) Application number: 01110765.3
(22) Date of filing: 03.05.2001
(51) Int. Cl.: G01N 33/543

(54) **Method for screening plant extracts for active ingredients**
Verfahren zum Screenen von Pflanzenextrakten nach Wirkstoffen
Méthode pour criblage les extraits végétaux pour agents actifs

(30) Priority: 07.11.2000 US 708306
(43) Date of publication of application: 15.05.2002
(73) Proprietor: Advanced Gene Technology, Corp., Taichung City (TW)
(72) Inventor: Hsu, Li-Wei, Taichung City (TW); Chang, Su-Chen, Taichung City (TW)
(74) Representative: Albrecht, Thomas, Dr.

(56) References cited:
- WO-A-01/09607
- WO-A-91/11458
- WO-A-99/38013
- AKIHISA T: "Alkane diols from flower petals of carthamus tinctorius." PHYTOCHEMISTRY, vol. 45, no. 4, 1997, pages 725-728, XP004293193
- AKIHSA T ET AL.: "Triterpene alcohols from the flowers of compositae and their anti-inflammatory effects." PHYTOCHEMISTRY, vol. 43, no. 4, 1997, pages 1255-1260, XP001071273

## Description

The present invention relates to the application of high-density gridding technology for the screening of the fractions of an extract of plant for biologically active ingredients of interest.

Plants, especially herbs, are natural sources of biologically active ingredients that have a pharmacological or therapeutic effect in animals. Many drugs or their precursors are isolated from plants. Normally, it is laborious to isolate a biologically active ingredient in a purified form from plant or its extract by applying conventional techniques, such as extraction, precipitation, centrifugation and complicated chromatographic technology. So far there is not a simple method for the screening of plant or its extract to select biologically active ingredients in a rapid manner.

Recently, the high-density gridding technology has been used in the art for qualitatively or quantitatively detecting the presence of a target material in biological samples. The high-density gridding technology immobilizes arrays of samples in a small or even tiny volume on a gridding surface of a solid support. In this technology, the biological samples suspected of containing target material are fixed or immobilized on a solid support, a labeled probe that can hybridize with or conjugate to the target material is added to the solid support, the hybridized or conjugated solid support is processed, imaged and analyzed, and the candidate target material that specifically interacts with the labeled probe can be quickly screened and selected.

However, so far the target material that can be successfully detected on a solid support by applying the high-density gndding technology is limited to several macromolecules, such as nucleic acid fragments/sequences or peptides or proteins. For instance, USP 6,004,755 disclosed a method for hybridization assay, comprising contacting an array of probe molecules stably associated with the surface of a solid support with an end labeled target nucleic acid sample under hybridization conditions for producing a hybridization pattern. USP 6,040,138 disclosed a method of hybridizing a nucleic acid sample to a high density array of oligonucleotide probes, in which the oligonucleotide probes in the high density array are complementary to the subsequences of target nucleic acids in the nucleic acid sample. USP 6,087,103 disclosed a method of screening a target for ligand binding, comprising using a tagged array of protein ligands bound to a solid support. It is not taught or suggested in the prior art or the state of the art that biologically active small molecules can be successfully screened and selected by using the high-density gridding technology.

There is a demand in the development of a rapid method for the screening of plant or its extract for biologically active small molecular components in a massive amount by using a simple process. In addition, to meet a technical requirement for the screening, there is also a demand in the development of a simple kit for the screening of plant or its extract for biologically active small molecular components.

WO 99/38013 discloses a method for the simultaneous identification of proteins and bonding partners characterized in that the invention relates to a method for simultaneous identification of a protein and its binding partners, characterised in that a) proteins or aggregates of proteins from a biological source are isolated and separated, b) the separated proteins or aggregates of proteins are immobilized on a surface, c) a combinatory bank is incubated with proteins or aggregates of proteins immobilized on a surface, d) the members of the combinatory bank which bond with the immobilized proteins are separated from non-bonded members of said bank, e) the surface bonded complexes made of protein and bonding partners from the combinatory bank are isolated, f) the proteins in the complexes thus isolated are identified according to a mixed physical-chemical process and g) the isolated bonding partners are optionally enriched. The invention enables simultaneous identification of proteins with or without prior purification, and makes it possible to select members of combinatory banks which interact with said proteins. This allows the function of said proteins to be determined in a simple manner, using their specific bonding partners.

WO 01/09607 which is a document under Article 54(3)(4) EPC discloses a fibre bundle comprising a plurality of fibres attached to each other in a fixed position with respect to each other wherein the fibres have different agents of interest immobilize in or on different fibres as well as an array comprising a plurality of said fibres.

Phytochemistry 45 (4): 725-728 (1997) discloses the isolation of alkane diols from flower petals of *Carthamus tinctorius*.

Phytochemistry 43 (6): 1255-1260 (1996) discloses the isolation of triterpene alcohols from the flowers of compositae and their anti-inflammatory effects.

WO 91/11458 relates to cyclic peptides containing Arg Gly Asp flamed by prolene as well as the use for reducing platelet aggregation in a mammal.

The present invention relates to a novel method for the screening of an extract of plant for biologically active ingredients having an inhibition activity in platelet aggregation or an in vivo antithrombotic activity, comprising fractionating a crude extract of plant on a solid support, adding on the support a labelled platelet membrane receptor protein gpIIb/IIIa that is capable of binding to said biologically active ingredients, and detecting and recovering the biologically active ingredients as well as to a kit for the screening of an extract of plant for biologically active ingredients having an inhibition activity in platelet aggregation or an in vivo antithrombotic activity, comprising a solid support on which fractions of an extract of plant are arrayed, a solution of a labelled platelet membrane receptor protein gpIIb/IIIa that is capable of binding to said biologically active ingredients, and reagents required for washing and signal detection for selecting and recovering the biologically active ingredients.

One embodiment of the present invention relates to the use of said novel method for obtaining a biologically active compound in a purified form from an extract of *Carthamus tinctorius L* that can specifically bind to a platelet membrane receptor protein gpIIb/IIIa.
Figure 1 A shows the HPLC elution profile of an extract of *Carthamus tinctorius L* in 120 minutes.
Figure 1B shows the binding profile of the eluted samples shown in Figure 1A to the protein gpIIb/IIIa that was detected by the absorption at a wavelength of 405 nm.
Figure 2A shows the HPLC elution profile of a sample pool collected from the fractions No. 25 to 40 shown in Figure 1A in 20 minutes.
Figure 2B shows the binding profile of the eluted samples shown in Figure 2A to the protein gpIIb/IIIa that was detected by the absorption at a wavelength of 405 nm.
Figure 3 shows the HPLC elution profile of a sample pool collected from the fractions No. 5 and 6 shown in Figure 2A in 15 minutes, indicating that there is a single peak with retention time of 10.7 minutes which shows the strongest binding activity to the protein gpIIb/IIIa.
Figure 4A shows the molecular weight 268 gm/mole of the compound obtained from the profile of Figure 3 that was detected by the electrospray mass spectrum.
Figure 4B shows the presence of sodium salt and oligomers (2-mer to 7-mer) of the compound obtained from the profile of Figure 3.
Figure 5 shows the binding curve of the compound obtained from the profile of Figure 3 with the protein gpIIb/IIIa, indicating that the binding dramatically increased up to 80 % of the maximum binding when the concentration of the compound is below 10 µg/ml.
Figure 6A shows a dose response of the compound obtained from the profile of Figure 3 in the inhibition of platelet aggregation, indicating that the maximum inhibition activity is about 85 % when the concentration of the compound is from 10 to 15 µg/ml blood.
Figure 6B shows the inhibition activity on platelet aggregation in a time course manner when the concentration of the compound obtained from the profile of Figure 3 is 17.4 µg/ml, indicating that about 85 % of the maximum inhibition activity occurs at a period from 14^{th} to 16^{th} minutes.
Figure 7A shows the inhibition of thrombosis formation *in vivo* in mesenteric vein of rats by administering the compound (400 µg/100 µl/rat) obtained from the profile of Figure 3 in a time course manner.
Figure 7B shows a dose response of the compound obtained from the profile of Figure 3 in the inhibition of thrombosis formation *in vivo* in mesenteric vein of rats by intravenous injection.
Figure 7C shows a dose response of the compound obtained from the profile of Figure 3 in the inhibition of thrombosis formation *in vivo* in mesenteric vein of rats by oral administration.

The present invention provides a rapid method for the screening of an extract of plant for biologically active ingredients. The method comprises fractionating a crude extract of plant, allocating the components in each fraction on a solid support, adding platelet membrane receptor protein gpIIb/IIIa as labeled target as a probe onto the solid support, incubating the solid support to allow the specific binding between biologically active ingredients of interest in the crude extract and the added probe, and then detecting and selecting the biologically active ingredients of interest.

It is well known in the art that plants, especially herbs, are treasures of natural sources for new drug discovery. Isolation of biologically active ingredients in a punfied form from plants by using the conventional techniques is laborious and time-consuming.

The high-density gridding technology has been used in the art for detecting a target material of interest in samples, which involves immobilizing arrays of samples in a small or even tiny volume on a gridding surface of a solid support. It is an advantage that the high-density gndding technology can perform hundreds or more of different experiments as in individual test tubes at the same time. By this technology, samples suspected of containing a target material of interest are fixed or immobilized on a solid support, a labeled probe that can hybridize with or conjugate to the target material is added to the solid support, the hybridized or conjugated solid support is processed, imaged and analyzed, and the candidate target material that specifically interacts with the labeled probe can be quickly detected. In practice, the high-density gridding technology has been used in the field of diagnosis, drug selection, discovery of therapeutic targets, and determination of pharmacological mechanism.

The target materials that can be successfully detected in the art by applying the high-density gridding technology are, however, limited to several macromolecules, such as fragments of nucleic acids or proteins. It is not known, taught or suggested in the art that small molecules exhibiting a biological activity of interest can be successfully detected and selected by applying the high-density gridding technology.

It is a discovery of the present invention that biologically active small molecules can be detected and isolated in a purified form from an extract of plant by applying the high-density gridding technology. According to the method of the present invention, a crude extract of plant is fractionated with the aid of chromatography, the components in individual fractions are immobilized on the surface of a gridding solid support, the solid support is then hybridized with a labeled target being platelet membrane receptor protein gpIIb/IIIa, the sites on the solid support containing the biologically active small molecules interacting with the labeled target are detected, and the biologically active small molecule is then recovered, isolated and finally purified.

In one embodiment of the present invention, an extract of herb was fractionated by HPLC in the beginning. Individual fractions are allocated on the surface of a gridding solid support. A labeled protein was added to the surface of the solid support for hybridization. The unbound protein was stripped off thereafter. The candidate fractions showing a signal of binding to the labeled protein were selected, and the previous steps are repeated until a single ingredient interacting with the labeled protein was obtained.

In one preferred embodiment of the present invention, an extract of *Carthamus tinctorius L* was fractionated by HPLC. Individual fractions were allocated on a plastic plate. A labeled platelet membrane receptor protein gpIIb/IIIa, an important factor capable of inhibiting platelet aggregation *in vitro* and thrombosis formation *in vivo*, was added to the plastic plate for binding. The unbound gpIIb/IIIa was stripped off. The candidate fractions showing a signal of binding to the labeled gpIIb/IIIa were selected, and the previous steps are repeated until a single ingredient interacting with the labeled gpIIb/IIIa was obtained.

The method of the present invention will be more efficient if integral automatic instruments for sampling, high-density arraying and detecting are used.

The present invention reveals the following advantages and improvement in effect: (i) the sample volume for screening can be minimized to a micro- or even pico-liter level when the samples are spotted onto the surface of a solid support, (ii) the presence of selected small molecules that directly interact with the labeled target clearly demonstrate that there is a specific binding between the small molecules and labeled target, and the screened small molecules may exhibit the desired pharmacological function, (iii) the solid support spotted with fractions of an extract of plant can be used as a chip of a specific plant, for instance as a herbal chip, that can be universally used as a platform for screening for a biologically active ingredient of interest, and (iv) the screening process can be preformed within a high density-spotted solid area instead of dealing with a large amount of reactions in test tubes.

The present invention will be further illustrated by the following examples; however, it should be noted that the present invention is not limited to the following examples.

### Examples

### Example 1

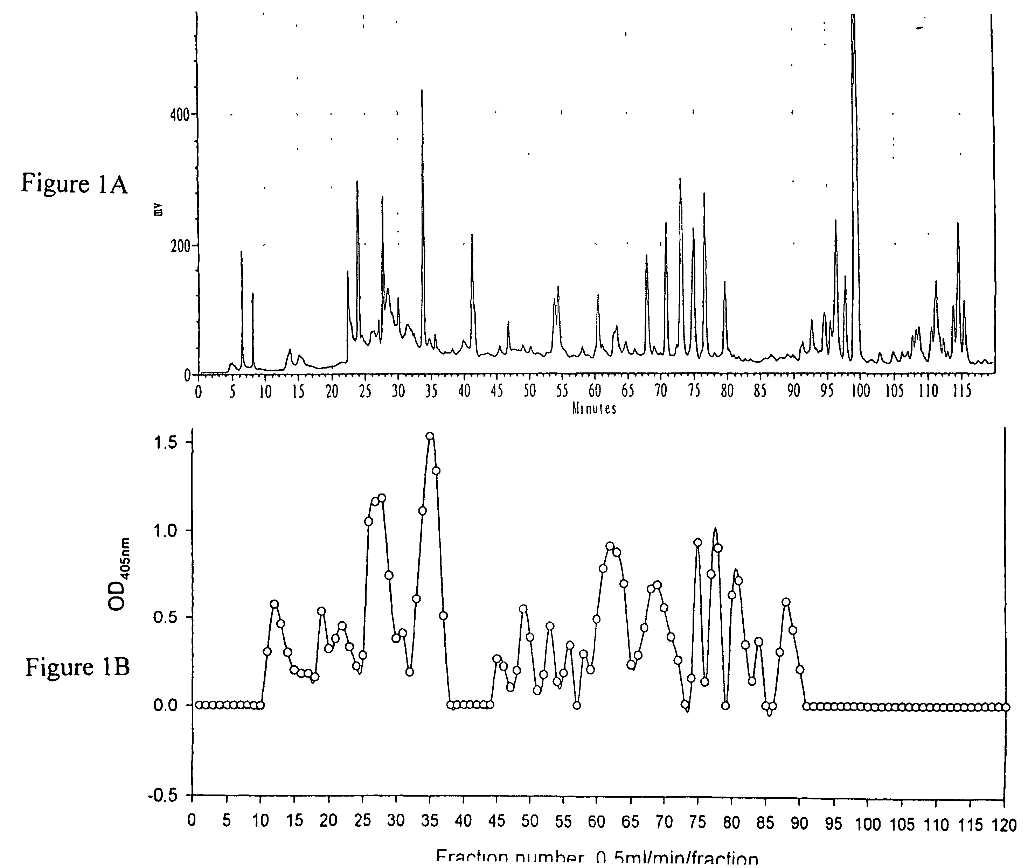

Five gram of *Carthamus tinctorius L* (purchased from Uni Chinese Herb Store, Taichung, Taiwan) was extracted with methanol (40 ml) by regular blending. The extract was concentrated to a final volume of 8 ml. For conducting HPLC (Shimadzu 10-AT, Japan) analysis, the concentrated extract (100 µl) was injected into ODS-gel ODS 80TM (4.6 mm × 25 cm, TOSOH, Japan) column. The concentrated extract was first eluted with water for 5 minutes, followed by elution with an ethanol-water eluant with a linear increase of ethanol concentration from 0 to 70 % (v/v) within 105 minutes. In the next 5 minutes for elution, the concentration of ethanol in the eluant was increased to 100 % (v/v). The eluted samples were detected at a wavelength of 254 nm and were collected every 0.5 ml. The elution profile was shown in Figure 1A.

For the target-binding assay, the platelet membrane receptor protein gpIIb/IIIa was purified from platelets, and its purity was determined by SDS-PAGE and silver staining. The purified protein gpIIb/IIIa was labeled with biotin as described in the conventional protocol. The 120 collected samples were individually coated onto a 384-well plastic plate. The biotin-labeled protein gpIIb/IIIa was added to the sample-coated plastic plate and was incubated with the coated samples at room temperature for 30 minutes. Each well was then washed with TBST buffer for 3 times, to which the extravidin-conjugated alkaline phosphatase was added, and each well was further incubated for 30 minutes. The previous washing step was repeated and each well was then colored with substrate p-nitrophenyl phosphate. The absorption for each well at a wavelength of 405 nm was determined by an automatic ELISA reader (Dynax). Figure 1B shows the OD₄₀₅ₙₘ value of the samples corresponding to those in Figure 1A, indicating their capacity of binding to the protein gpIIb/IIIa.

### Example 2

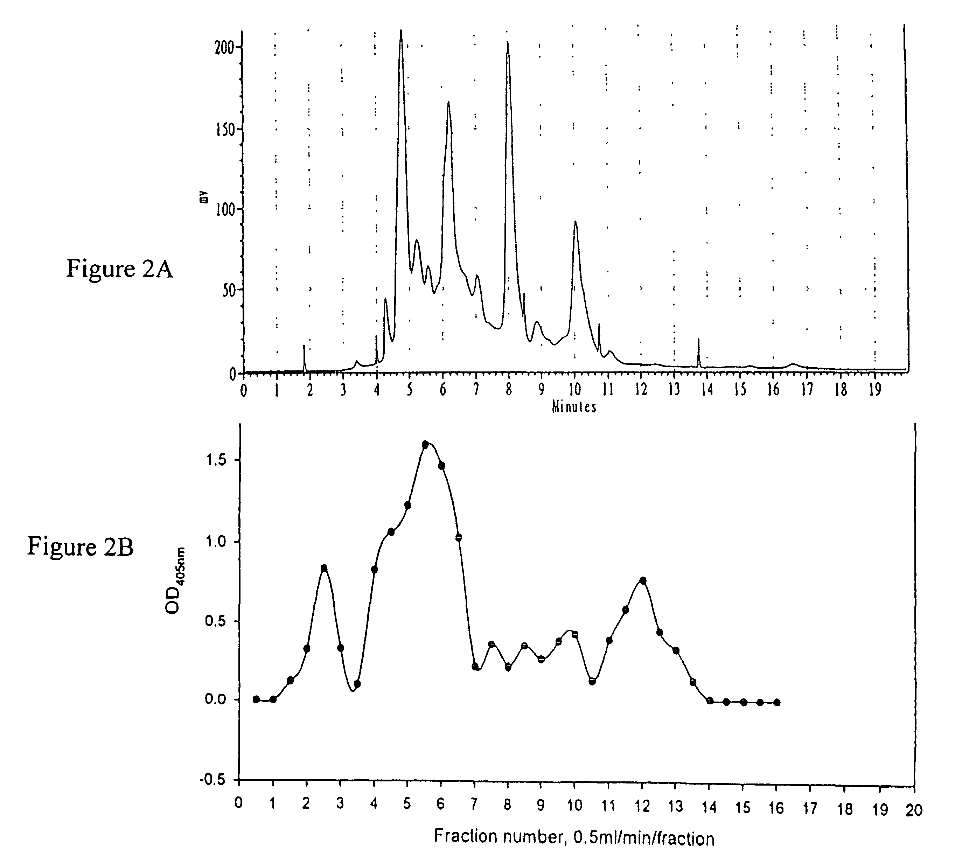

The fractions corresponding to No. 25 to 40 in Figure 1A were pooled together, and were injected into the column and analyzed by HPLC in a manner similar to that described in Example 1. The ethanol-water elution was changed to a 20-minute program with a linear increase of ethanol concentration from 20 to 30 %. The elution profile is shown in Figure 2A.

The target protein gpIIb/IIIa-binding assay was performed in a way as that described in Example 1. The result indicating the capacity of gpIIb/IIIa binding for each fraction is shown in Figure 2B.

### Example 3

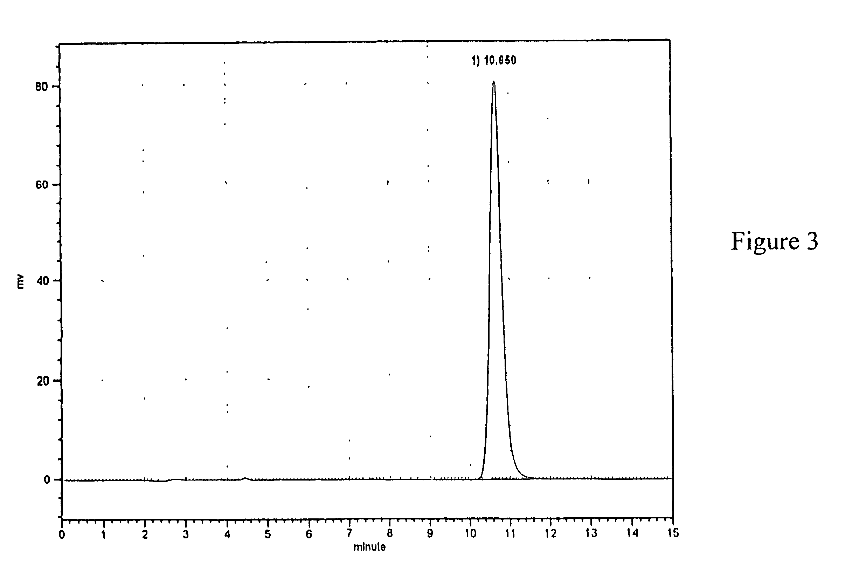

The fraction 5 and 6 shown in Figure 2A were pooled together, on which there was run a third round HPLC analysis with an isocratic eluant of 9 % ethanol and 91 % water for 20 minutes. A single peak with retention time of 10.7 minute showing the strongest binding activity is obtained. The refining profile is shown in Figure 3.

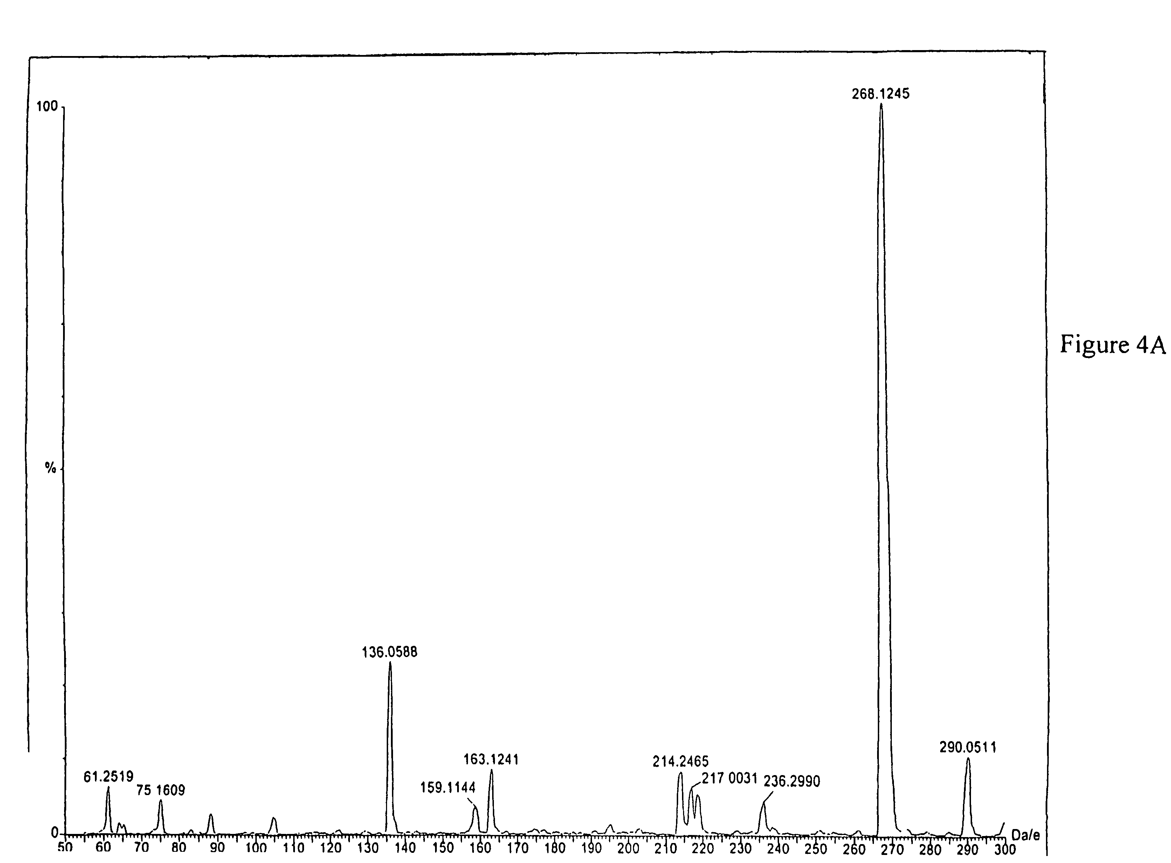

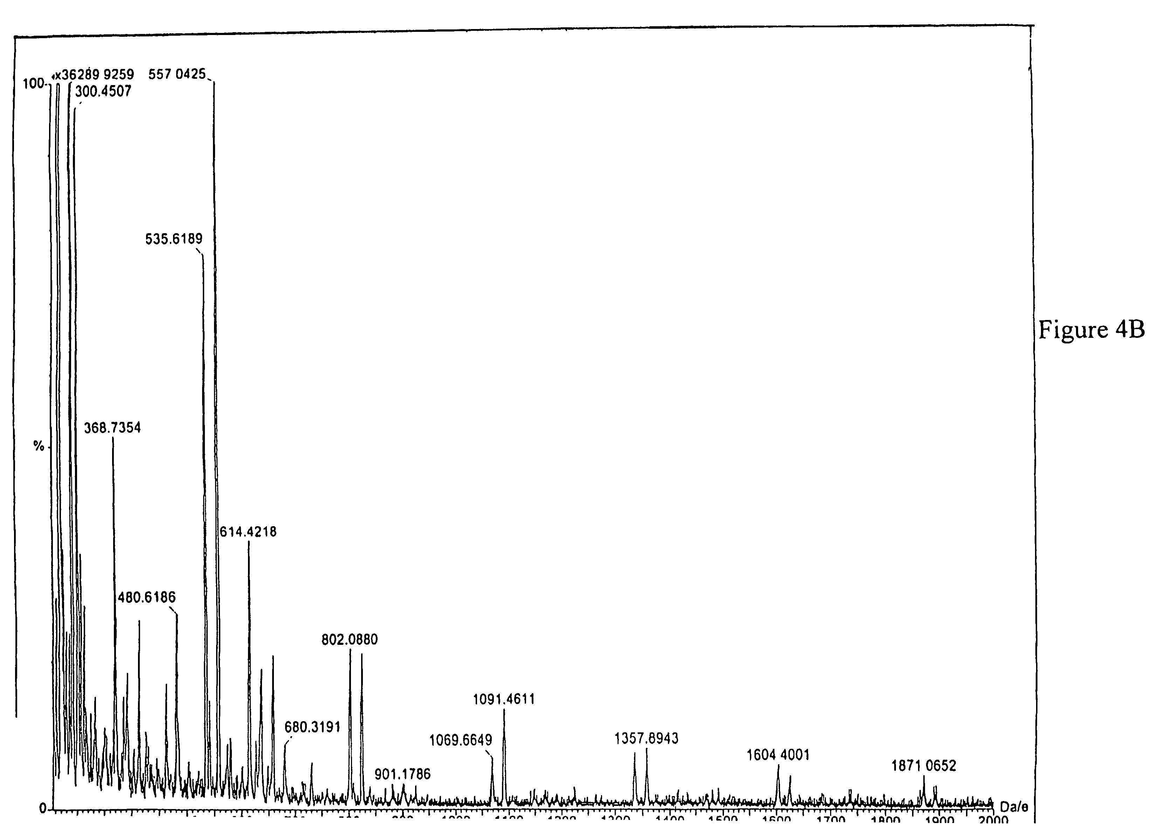

The compound collected from the profile of Figure 3 was dried, and its molecular weight (MW) was determined by the electrospray mass spectrum. As shown in Figure 4A, the major peak was determined to be an m/z of 268.04 with a standard deviation of 0.11. Another 289.9 m/z paired peak was also determined by approximately 21.9 m/z apart. It was proposed to be a sodium salt of the major peak. Furthermore, a number of related, multiply-charged and paired peaks (535.6; 802.1; 1069.7; 1357.9; 1604.4; and 1871.1 m/z) were also determined (Figure 4B). Multiply-charged electrospray is often determined for biopolymers, and those peaks shown in Figure 4B were exactly correspondent to dimmer to 7-mer, respectively. A paired signal by approximately 22 m/z apart associated with each multiplied peak was proposed to be its conjugated sodium salt. In conclusion, the isolated compound has molecular weight of 268 gm/mole and exhibits a polymer-forming capacity.

### Example 4

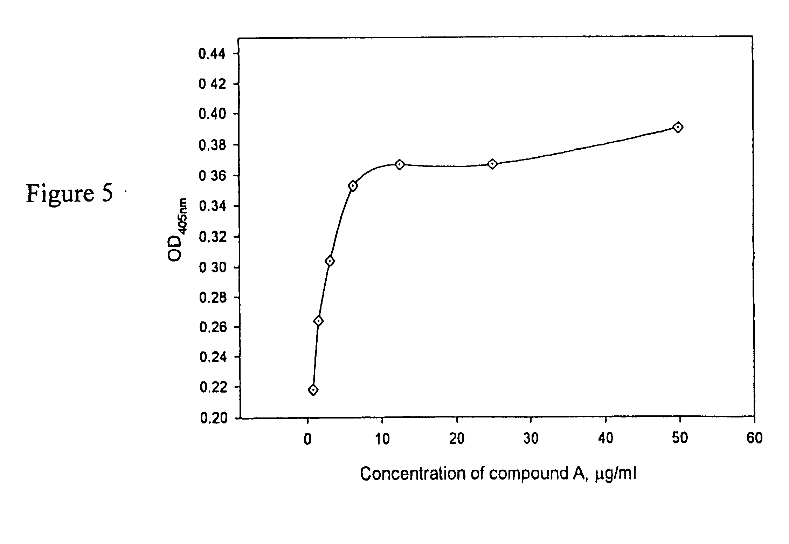

The profile shown in Figure 3 was used as a standard for the characterization of each batch preparation of the compound. The purified compound was collected, dried and used to verify its gpIIb/IIIa binding capacity. For conducting the binding assay, the purified compound was prepared in an aqueous solution, and a serial dilution for forming a final concentration of 0 to 50 µg/ml is made. Each tested solution contained a different amount of the compound, and was spotted on a flat well of a plastic 96-well plate. The labeled protein gpIIb/IIIa was then added into each tested well, and the coloring process was performed in a way as previously described. The binding curve was shown in Figure 5. It was observed that the binding dramatically increased up to 80 % of the maximal binding with the concentration below 10 µg/ml.

### Example 5

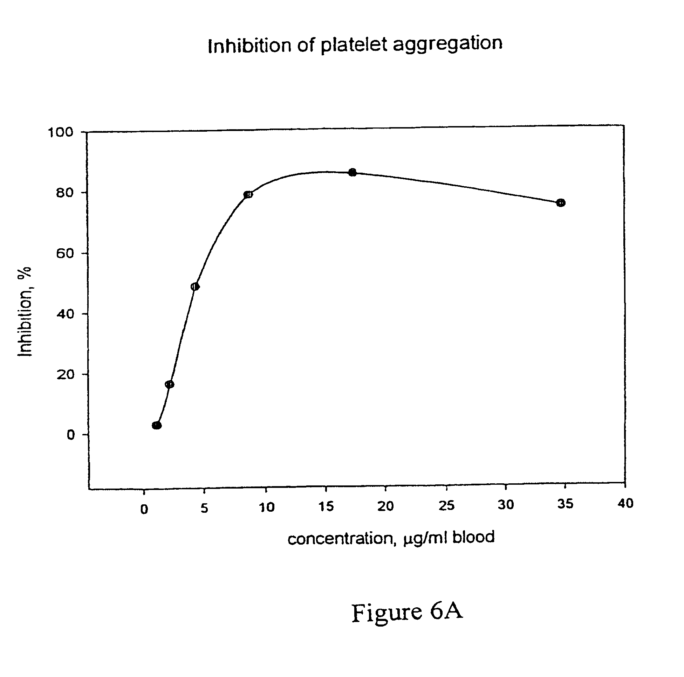

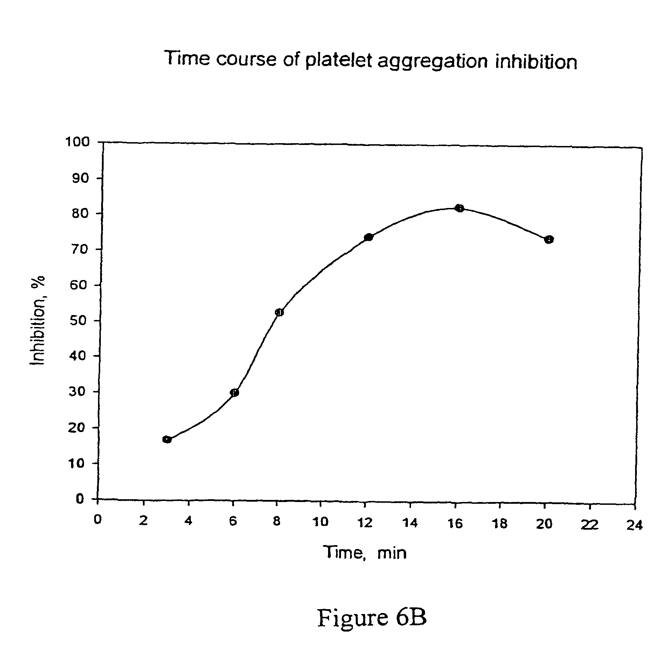

To assay the platelet aggregation-inhibiting activity of the compound, a regular ADP-activated platelet aggregation assay was performed. The reagents used for conducting the assay were purchased from Sigma. Figure 6A showed a dose response of the compound in the inhibition of platelet aggregation. The maximal inhibition activity was about 85 %, which occurs at the concentration of the compound from 10 to 15 µg/ml blood. By using the compound with the concentration of 17.4 µg/ml for determining the inhibition activity on platelet aggregation in a time course manner, Figure 6B revealed that the maximal inhibition activity was about 85 % occurring at the time from the 14^{th} to 16^{th} minutes.

### Example 6

The thrombosis formation *in vivo* in mesenteric vein of rats was used for evaluating an effect of the compound on the inhibition of venous thrombosis. In brief, groups of 3 male Wistar-denved rats, each weighing 60 ± 10 gm, were used for each dosage test. The rats were anesthetized with phenobarbital sodium (50 mg/kg, i.p.), and paralyzed with succinylcholine chloride (2 mg/rat, i.p.). A mesenteric loop and vein was then exposed and mounted on a constructed platform. The exposed area was superfused with a normal saline at 37 °C, except during electrode placement and stimulation. With the aid of a dissecting microscope and micromanipulator, a monopolar platinum electrode was brought into and contact with the vein. Thrombus formation was initiated by the application of a single square wave electrical pulse (1000PPS, 100V, 300ms) supplied from a grass S-44 stimulator. The formation of thrombus was then observed through the microscope-calibrated ocular lens. The relative venous occlusion (a measured degree of thrombus formation) was determined as a percentage of venous diameter (0.36 to 0.38mm) and was recorded at 10 seconds (baseline control score) and at 1 minute interval for 20 minutes. The tested compound or vehicle was administered i.v. for 5 minutes before conducting the electrical stimulation of mesenteric vein. In the vehicle-treated animals, the 20 interval recorded values were averaged, which attains a value ranging from 45 to 55% of the interval venous diameter. Consequently, the antithrombic activity of tested compound was calculated as % of inhibition relative to the vehicle-treated control animals. If a significant inhibition (> 30%) was observed in 3 animals, an ED₃₀ ± SEM was determined by linear regression using 3 animals per dose level. At each time point, a paired Student's *t* test was applied for the statistical analysis for comparison of the vehicle treatment group with the tested compound group with a significance referred to as *P < 0.05 and **P < 0.01.

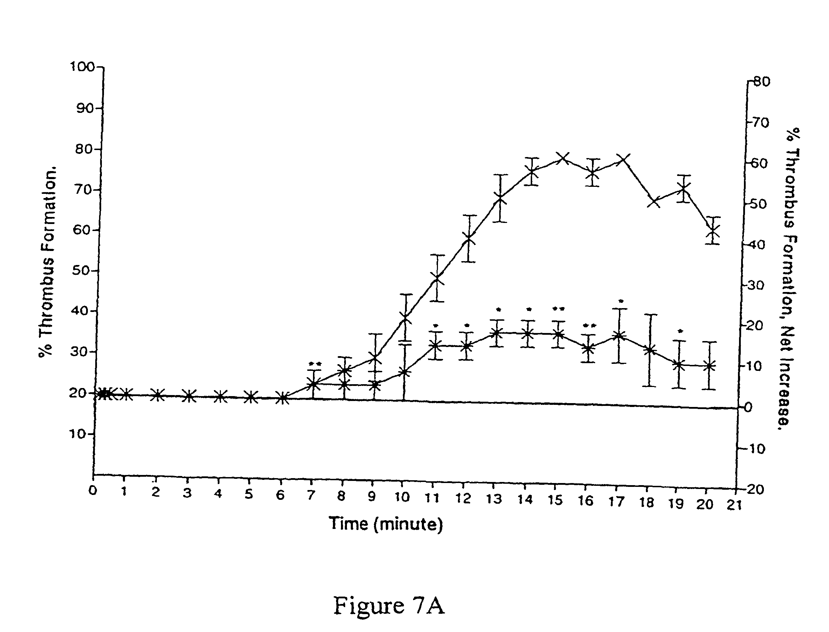

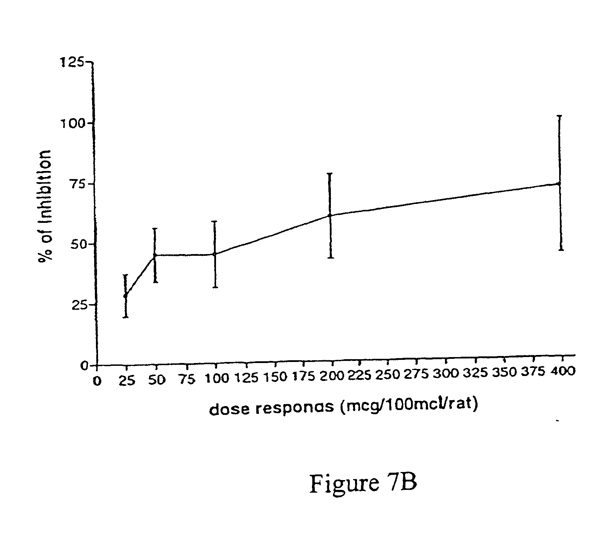

In Figure 7A, the compound exhibiting an antithrombic activity with a concentration of 400 µg/rat revealed significance relative to the vehicle treated group at 7, 11,12, 13, 14, 15, 16, 17, and 19 minutes after inducing an electric stimulation. The antithrombic activity of the tested compound in a dose response manner in a range of 25, 50, 100, 200 and 400 µg/rat was shown in Figure 7B.

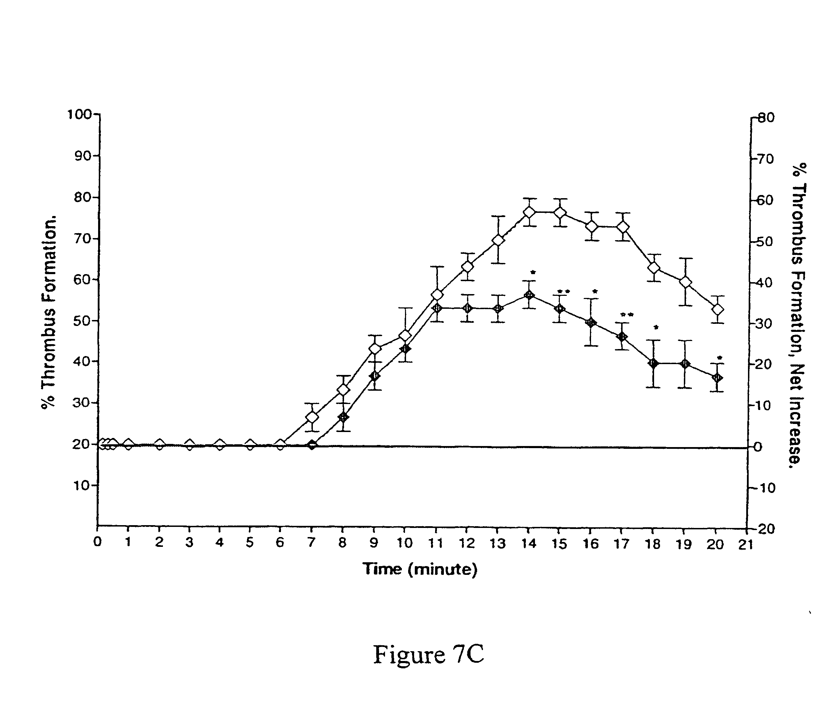

For evaluating the antithrombic activity of the tested compound by oral administration, an animal model experiment was similarly performed, except that the intravenous injection is replaced with oral feeding at 1 hour prior to the electrical stimulation. A dosage amount of 4 mg/rat was administered. The result was shown in Figure 7C, which revealed that the significant antithrombic activity relative to the vehicle treated group was observed at 14, 15, 16, 17, 18, and 20 minutes.

## Claims

1. A method for the screening of an extract of plant for biologically active ingredients having an inhibition activity in platelet aggregation or an in vivo antithrombotic activity, comprising fractionating a crude extract of plant on a solid support, adding on the support a labelled platelet membrane receptor protein gpIIb/IIIa that is capable of binding to said biologically active ingredients, and detecting and recovering the biologically active ingredients.

2. The method of claim 1, wherein the plant is an herb.

3. The method of claim 2, wherein the herb is *Carthamus tinctorius L.*

4. A kit for the screening of an extract of plant for biologically active ingredients having an inhibition activity in platelet aggregation or an in vivo antithrombotic activity, comprising a solid support on which fractions of an extract of plant are arrayed, a solution of a labelled platelet membrane receptor protein gpIIb/IIIa that is capable of binding to said biologically active ingredients, and reagents required for washing and signal detection for selecting and recovering the biologically active ingredients.

5. The kit of claim 4, wherein the plant is an herb.

6. The kit of claim 4, wherein the herb is *Carthamus tinctorius L.*

## Patentansprüche

1. Verfahren fur das Screenen eines Pflanzenextrakts nach biologisch aktiven Bestandteilen, die eine Inhibitionsaktivitat bei der Plattchenaggregation oder eine antithrombotische in vivo-Aktivitat aufwelsen, umfassend Fraktionieren eines Pflanzenrohextrakts auf einem festen Trager, Zugeben eines markierten Plattchenmembranrezeptorproteins gpIIb/IIIa, das in der Lage ist, an die biologisch aktiven Bestandteile zu binden, zu dem Trager, und Nachweisen und Ruckgewinnen der biologisch aktiven Bestandteile.

2. Verfahren nach Anspruch 1, wobei die Pflanze ein Kraut ist.

3. Verfahren nach Anspruch 2, wobei das Kraut *Carthamus tinctorius L.* ist.

4. Kit fur das Screenen eines Pflanzenextrakts nach biologisch aktiven Bestandteilen, die eine Inhibitionsaktivitat bei der Plattchenaggregation oder eine antithrombotische in vivo-Aktivitat aufweisen, umfassend einen festen Trager, auf welchem Fraktionen eines Pflanzenextrakts angeordnet sind, eine Losung eines markierten Plattchenmembranrezeptorproteins gpIIb/IIIa, das in der Lage ist, an die biologisch aktiven Bestandteile zu binden, und Reagenzien, erforderlich fur Waschen und Signalnachweis fur das Auswahlen und Ruckgewinnen der biologisch aktiven Bestandteile.

5. Kit nach Anspruch 4, wobei die Pflanze ein Kraut ist.

6. Kit nach Anspruch 4, wobei das Kraut *Carthamus tinctorius L.* ist.

## Revendications

1. Procédé de criblage d'un extrait de plante quant à la présence d'ingrédients biologiquement actifs possédant un effet inhibiteur sur l'agrégation plaquettaire ou un effet antithrombotique *in vivo*, comprenant le fractionnement d'un extrait brut de plante sur un support solide, l'adjonction au support d'une protéine de récepteur de membrane de plaquette gpIIb/IIIa marquée qui est capable de se lier aux dits ingrédients biologiquement actifs, et la détection et la récupération des ingrédients biologiquement actifs.

2. Procédé de la revendication 1, dans lequel la plante est une herbe.

3. Procédé de la revendication 2, dans lequel l'herbe est *Carthamus tinctorius L.*

4. Kit de criblage d'un extrait de plante quant à la présence d'ingrédients biologiquement actifs possédant un effet inhibiteur sur l'agrégation plaquettaire ou un effet antithrombotique *in vivo*, comprenant un support solide sur lequel sont réparties des fractions d'un extrait de plante, une solution d'une protéine de récepteur de membrane de plaquette gpIIb/IIIa marquée qui est capable de se lier aux dits ingrédients biologiquement actifs, et les réactifs requis pour le lavage et la détection d'un signal pour la sélection et la récupération des ingrédients biologiquement actifs.

5. Kit de la revendication 4, dans lequel la plante est une herbe.

6. Kit de la revendication 4, dans lequel l'herbe est *Carthamus tinctorius L.*
